Europäisches Patentamt

(19) European Patent Office

Office européen des brevets

(11) **EP 1 518 493 B1**

(12) **EUROPEAN PATENT SPECIFICATION**

(45) Date of publication and mention
of the grant of the patent:
**11.10.2006 Bulletin 2006/41**

(51) Int Cl.:
*A61B 5/00* (2006.01)        *G01K 3/10* (2006.01)
*G01K 13/00* (2006.01)

(21) Application number: **04001496.1**

(22) Date of filing: **23.01.2004**

(54) **Blood sugar level measuring method**

Vorrichtung zur Messung des Blutzuckergehalts

Appareil de mesure du taux de glycémie

(84) Designated Contracting States:
**DE FR GB**

(30) Priority: **29.09.2003 JP 2003338580**

(43) Date of publication of application:
**30.03.2005 Bulletin 2005/13**

(73) Proprietor: **Hitachi, Ltd.**
**Chiyoda-ku,**
**Tokyo 100-8010 (JP)**

(72) Inventors:
• **Cho, Ok-Kyung**
**58239 Schwerte (DE)**

• **Kim, Yoon-Ok**
**58239 Schwerte (DE)**

(74) Representative: **Strehl Schübel-Hopf & Partner**
**Maximilianstrasse 54**
**80538 München (DE)**

(56) References cited:
EP-A- 0 778 000        EP-A- 1 484 006
WO-A-01/28414        US-A- 5 732 711
US-A1- 2002 183 646        US-A1- 2003 152 133

**Description**

BACKGROUND OF THE INVENTION

Field of the Invention

The present invention relates to an apparatus for non-invasively measuring glucose concentration in a living body without blood sampling.

Background Art

Hilson *et al.* report facial and sublingual temperature changes in diabetics following intravenous glucose injection (Non-patent literature 1). Scott *et al.* discuss the issue of diabetics and thermoregulation (Non-patent literature 2). Based on such researches, Cho *et al.* suggest a method and apparatus for determining blood glucose concentration by temperature measurement without requiring the collection of a blood sample (Patent literature 1, 2 and 6).
Various other attempts have been made to determine glucose concentration without blood sampling. For example, a method has been suggested (patent literature 3) whereby a measurement site is irradiated with near-infrared light of three wavelengths, and the intensity of transmitted light as well as the temperature of the living body is detected. Then, representative values of the second-order differentiated values of absorbance are calculated, and the representative values are corrected in accordance with the difference between the living body temperature and a predetermined reference temperature. The blood sugar level corresponding to the thus corrected representative values is then determined. An apparatus is also provided (patent literature 4) whereby a measurement site is heated or cooled while monitoring the living body temperature. The degree of attenuation of light based on light irradiation is measured at the moment of temperature change so that the glucose concentration responsible for the temperature-dependency of the degree of light attenuation can be measured. Further, an apparatus is reported (patent literature 5) whereby an output ratio between reference light and the light transmitted by an irradiated sample is taken, and then the glucose concentration is calculated by a linear expression of the logarithm of the output ratio and the living body temperature.

(Non-patent literature 1)
R.M. Hilson and T.D.R. Hockaday, "Facial and sublingual temperature changes following intravenous glucose injection in diabetics," Diabete & Metabolisme, 8, pp.15-19: 1982
(Non-patent literature 2)
A.R. Scott, T. Bennett, I.A. MacDonald, "Diabetes mellitus and thermoregulation," Can. J. Physiol. Pharmacol., 65, pp. 1365-1376: 1987
(Patent Literature 1)
U.S. Patent No. 5,924,996
(Patent Literature 2)
U.S. Patent No. 5,795,305
(Patent Literature 3)
JP Patent Publication (Kokai) No. 2000-258343 A
(Patent Literature 4)
JP Patent Publication (Kokai) No. 10-33512 A (1998)
(Patent Literature 5)
JP Patent Publication (Kokai) No. 10-108857 A (1998)
(Patent Literature 6)
WO 01/28414 A2 (The pre-characterising part of claim 1 starts out from the prior art disclosed in this document.)

SUMMARY OF THE INVENTION

Glucose (blood sugar) in blood is used for glucose oxidation reaction in cells to produce necessary energy for the maintenance of living bodies. In the basal metabolism state, in particular, most of the produced energy is converted into heat energy for the maintenance of body temperature. Thus, it can be expected that there is some relationship between blood glucose concentration and body temperature. However, as is evident from the way sicknesses cause fever, the body temperature also varies due to factors other than blood glucose concentration. While methods have been proposed to determine blood glucose concentration by temperature measurement without blood sampling, they lack sufficient accuracy.
It is the object of the invention to provide an apparatus for determining blood glucose concentration with high accuracy based on temperature data of a subject without blood sampling. This object is solved by an apparatus according to claim 1. The dependent claims relate to preferred embodiments of the invention.

Blood sugar is delivered to the cells throughout the human body via the blood vessel system, particularly the capillary blood vessels. In the human body, complex metabolic pathways exist. Glucose oxidation is a reaction in which, fundamentally, blood sugar reacts with oxygen to produce water, carbon dioxide, and energy. Oxygen herein refers to the oxygen delivered to the cells via blood. The amount of oxygen supply is determined by the blood hemoglobin concentration, the hemoglobin oxygen saturation, and the amount of blood flow. On the other hand, the heat produced in the body by glucose oxidation is dissipated from the body by convection, heat radiation, conduction, and so on. On the assumption that the body temperature is determined by the balance between the amount of energy produced in the body by glucose burning, namely heat production, and heat dissipation such as mentioned above, we set up the following model:

(1) The amount of heat production and the amount of heat dissipation are considered equal.
(2) The amount of heat production is a function of the blood glucose concentration and the amount of oxygen supply.
(3) The amount of oxygen supply is determined by the blood hemoglobin concentration, the blood hemoglobin oxygen saturation, and the amount of blood flow in the capillary blood vessels.
(4) The amount of heat dissipation is mainly determined by heat convection and heat radiation.

According to this model, we achieved the present invention after realizing that blood sugar levels can be accurately determined on the basis of the results of measuring the temperature of the body surface and the blood oxygen concentration and the blood flow amount. The parameters can be measured from a part of the human body, such as the fingertip. Parameters relating to convection and radiation can be determined by carrying out thermal measurements on the fingertip. Parameters relating to blood hemoglobin concentration and blood hemoglobin oxygen saturation can be determined by spectroscopically measuring the blood hemoglobin and then finding the ratio between the hemoglobin bound with oxygen and the hemoglobin not bound with oxygen. The parameter relating to the amount of blood flow can be determined by measuring the amount of heat transfer from the skin.

The state of the measuring apparatus or that of the examined portion relative to the measuring apparatus is monitored, and the measurement is reset if there is an error, thus ensuring a reliable measurement.

The following describes a method for measuring blood sugar level using a measuring apparatus in accordance with an embodiment of the invention. The method comprises a first step of determining whether or not the output from the temperature detecting portion is within a predetermined range when the body surface is not in contact with the body-surface contact portion, and a second step of continuing with measurement if the output is within the predetermined range but making an error display and resetting the measurement if said output is outside the predetermined range. The temperature detecting portion may comprise a radiation temperature detector for detecting radiation temperature, the detector having an open end thereof positioned at the body-surface contact portion. In the first step, comparisons may be made between the detected radiation temperature and a predetermined radiation temperature threshold, between the detected environment temperature and a predetermined environment temperature, and between information about the amount of change in the temperature measured on the body surface and a predetermined threshold.

The further blood sugar level measuring method comprises a step of detecting chronological change in the output from the temperature detecting portion, detecting the presence or absence of contact of a body surface to the body-surface contact portion based on the chronological change in the output, a first step of starting the storage of the measurement data for the calculation of the blood sugar level upon detection of contact of the body surface to the body surface contact portion, a second step of detecting the moment of departure of the body surface from the body surface contact portion based on chronological changes in the output from the temperature detecting portion, and a third step of making an error display and resetting the measurement if the detected moment is within a predetermined time, or if the departure of the body surface from the body surface contact portion is not detected a certain time after the predetermined time. The blood sugar level measuring method further includes the step of making an advance-notice display of the timing of departure of the body surface from the body surface contact portion. In this case, in the third step, the predetermined time is the time between the time of start of storage of the measurement data and the timing of departure of the body surface. The advance-notice of the timing of departure of the body surface from the body surface contact portion may be made by way of a countdown to the timing on a display portion of the measuring apparatus.

BRIEF DESCRIPTION OF THE DRAWINGS

Fig. 1 shows a model of heat transfer from the body surface to a block.
Fig. 2 plots the measurement values of temperatures $T_1$ and $T_2$ as they change with time.
Fig. 3 shows an example of measuring the chronological change in temperature $T_3$.

Fig. 4 shows the relationships between measurement values provided by various sensors and the parameters derived therefrom.

Fig. 5 shows an upper plan view of a non-invasive blood sugar level measuring apparatus according to an embodiment of the present invention.

Fig. 6 shows the details of chronological change in the temperatures $T_1$ and $T_2$.

Fig. 7 shows the details of chronological change in the temperature $T_3$.

Fig. 8 illustrates the timing of placement of the finger.

Fig. 9 shows the operating procedure for the apparatus.

Fig. 10 shows the measuring portion in detail.

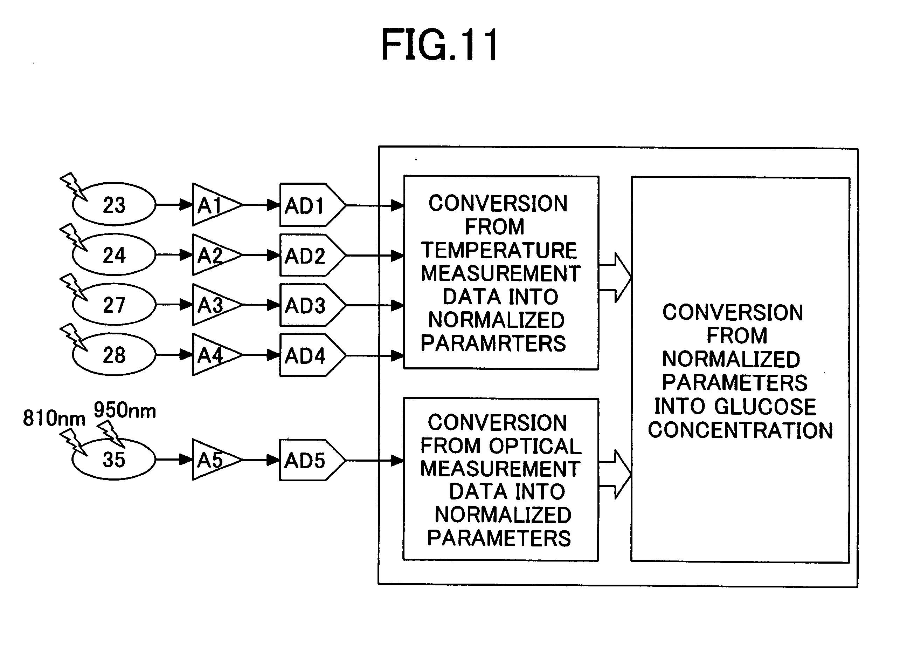

Fig. 11 shows a conceptual chart illustrating the flow of data processing in the apparatus.

Fig. 12 shows an example of the configuration inside the apparatus.

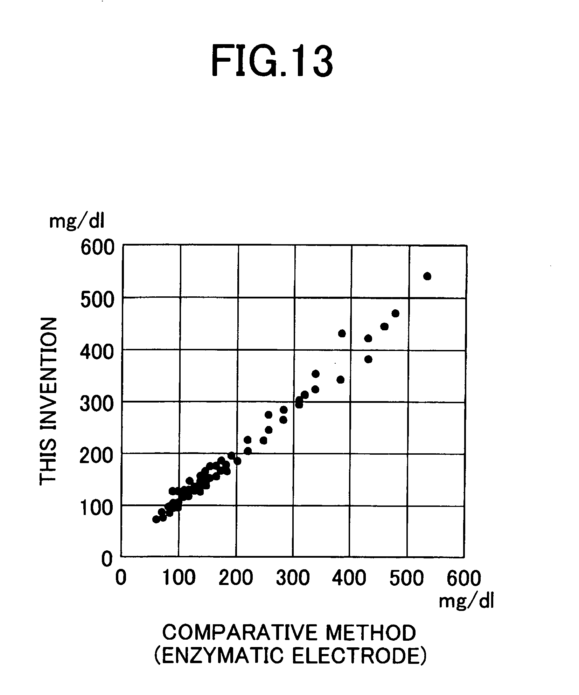

Fig. 13 shows the plots of the glucose concentration values calculated according to the present invention and the glucose concentration values measured by the enzymatic electrode method.

## DESCRIPTION OF THE PREFERRED EMBODIMENTS

The invention will now be described by way of preferred embodiments thereof with reference made to the drawings.

Initially, the above-mentioned model will be described in more specific terms. Regarding the amount of heat dissipation, convective heat transfer, which is one of the main causes of heat dissipation, is related to temperature difference between the ambient (room) temperature and the body-surface temperature. The amount of heat dissipation due to radiation, another main cause of dissipation, is proportional to the fourth power of the body-surface temperature according to the Stefan-Boltzmann law. Thus, it can be seen that the amount of heat dissipation from the human body is related to the room temperature and the body-surface temperature. Another major factor related to the amount of heat production, oxygen supply, is expressed as the product of hemoglobin concentration, hemoglobin oxygen saturation, and blood flow volume.

The hemoglobin concentration can be measured from the absorbance at a wavelength (equal-absorbance wavelength) at which the molar absorbance coefficient of the oxi-hemoglobin is equal to that of the deoxi-hemoglobin. The hemoglobin oxygen saturation can be measured by measuring the absorbance at the equal-absorbance wavelength and the absorbance at at least one different wavelength at which the ratio between the molar absorbance coefficient of the oxi-hemoglobin and that of the deoxi-hemoglobin is known, and then solving simultaneous equations. Namely, the hemoglobin concentration and the hemoglobin oxygen saturation can be obtained by measuring absorbance at at least two wavelengths.

The rest is the blood flow volume, which can be measured by various methods. One example will be described below.

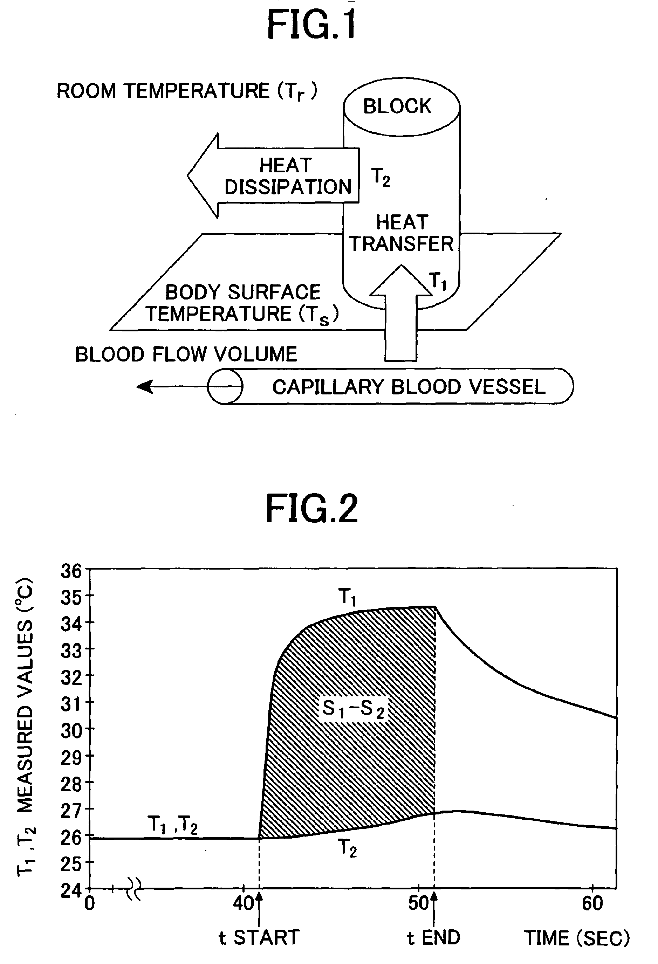

Fig. 1 shows a model for the description of the transfer of heat from the body surface to a solid block with a certain heat capacity as the block is brought into contact with the body surface for a certain time and then separated. The block is made of resin such as plastic or vinyl chloride. In the illustrated example, attention will be focused on the chronological variation of a temperature $T_1$ of a portion of the block in contact with the body surface, and the chronological variation of a temperature $T_2$ of a point on the block away from the body surface. The blood flow volume can be estimated by monitoring mainly the chronological variation of the temperature $T_2$ (at the spatially distant point on the block). The details will be described later.

Before the block comes into contact with the body surface, the temperatures $T_1$ and $T_2$ at the two points of the block are equal to the room temperature $T_r$. When a body-surface temperature $T_s$ is higher than the room temperature $T_r$, the temperature $T_1$ swiftly rises due to the transfer of heat from the skin as the block contacts the body surface, and it approaches the body-surface temperature $T_s$. On the other hand, the temperature $T_2$ becomes less than the temperature $T_1$ as the heat conducted through the block is dissipated from the block surface, and it rises more gradually than the temperature $T_1$. The chronological variation of the temperatures $T_1$ and $T_2$ depends on the amount of heat transferred from the body surface to the block, which in turn depends on the blood flow volume in the capillary blood vessels under the skin. If the capillary blood vessels are regarded as a heat exchanger, the heat transfer coefficient from the capillary blood vessels to the surrounding cell tissues is given as a function of the blood flow volume. Thus, by measuring the amount of heat transfer from the body surface to the block by monitoring the chronological variation of the temperatures $T_1$ and $T_2$, the amount of heat transmitted from the capillary blood vessels to the cell tissues can be estimated, which in turn makes it possible to estimate the blood flow volume can be estimated. Accordingly, by monitoring the temperature changes in $T_1$ and $T_2$ chronologically and measuring the amount of heat transfer from the body surface to the block, the amount of heat transfer from the capillary blood vessels to the cell tissues can be estimated, which in turn makes it possible to estimate the blood flow volume.

Fig. 2 shows the chronological variation of the measured values of the temperature $T_1$ at the portion of the block in contact with the body surface and the temperature $T_2$ on the block away from the body-surface contact position. As the block comes into contact with the body surface, the $T_1$ measured value swiftly rises, and it gradually drops as the block is brought out of contact.

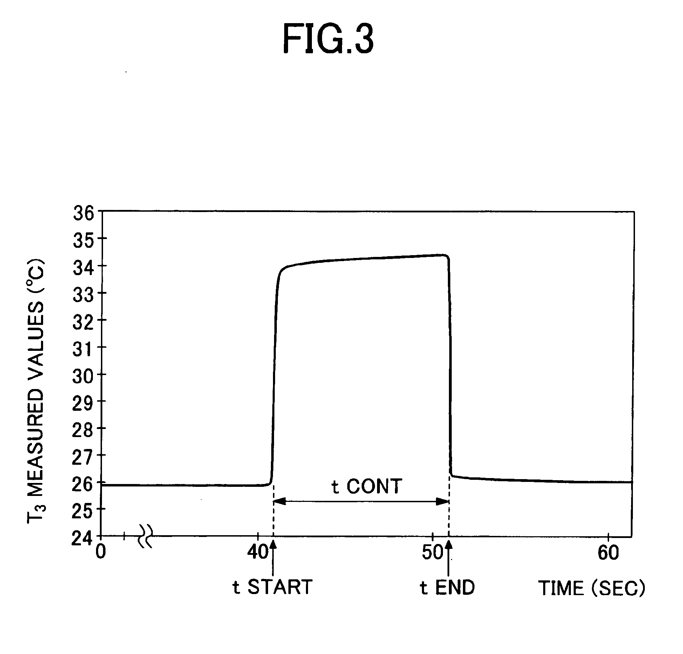

Fig. 3 shows the chronological variation of the measured value of the temperature $T_3$ measured by a radiation temperature detector. As the detector detects the temperature due to the radiation from the body surface, it is more sensitive to temperature changes than other sensors. Because radiation heat propagates as an electromagnetic wave, it can transmit temperature changes instantaneously.

Then, the $T_1$ measured value between $t_{start}$ and $t_{end}$ is approximated by an S curve, such as a logistic curve. A logistic curve is expressed by the following equation:

$$T = \frac{b}{1 + c \times \exp(-a \times t)} + d$$

where T is temperature, and t is time.

The measured value can be approximated by determining factors a, b, c, and d by the non-linear least-squares method. For the resultant approximate expression, T is integrated between time $t_{start}$ and time $t_{end}$ to obtain a value $S_1$.

Similarly, an integrated value $S_2$ is calculated from the $T_2$ measured value. The smaller the $(S_1 - S_2)$ is, the larger the amount of transfer of heat from the finger surface to the position of $T_2$. $(S_1 - S_2)$ becomes larger with increasing finger contact time $t_{cont}$ (=$t_{end} - t_{start}$). Thus, $e_5/(t_{cont} \times (S_1 - S_2))$ is designated as a parameter $X_5$ indicating the amount of blood flow, where $e_5$ is a proportionality coefficient.

Thus, it will be seen that the measured quantities necessary for the determination of blood glucose concentration by the above-described model are the room temperature (ambient temperature), body surface temperature, temperature changes in the block in contact with the body surface, the temperature due to radiation from the body surface, and the absorbance at at least two wavelengths.

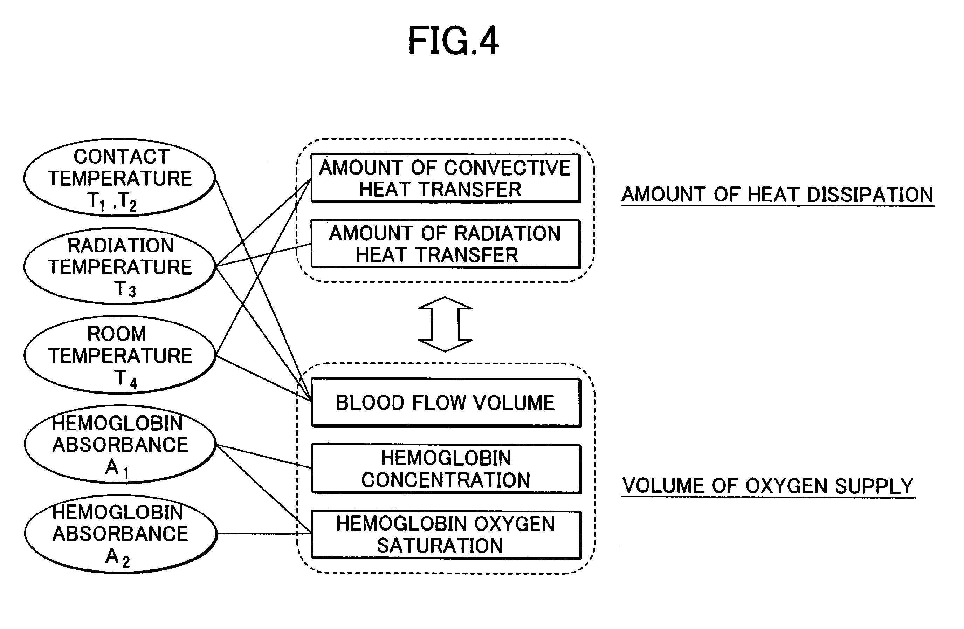

Fig. 4 shows the relationships between the measured values provided by various sensors and the parameters derived therefrom. A block is brought into contact with the body surface, and chronological changes in two kinds of temperatures $T_1$ and $T_2$ are measured by two temperature sensors provided at two locations of the block. A radiation temperature $T_3$ on the body surface and the room temperature $T_4$ are separately measured. Absorbance $A_1$ and $A_2$ are measured at at least two wavelengths related to the absorption of hemoglobin. The temperatures $T_1$, $T_2$, $T_3$, and $T_4$ provide parameters related to the amount of blood flow. The temperature $T_3$ provides a parameter related to the amount of heat transferred by radiation. The temperatures $T_3$ and $T_4$ provide parameters related to the amount of heat transferred by convection. The absorbance $A_1$ provides a parameter related to hemoglobin concentration. The absorbance $A_1$ and $A_2$ provide parameters related to the hemoglobin oxygen saturation.

Hereafter, an example of apparatus for non-invasively measuring blood sugar levels according to the principle of the invention will be described.

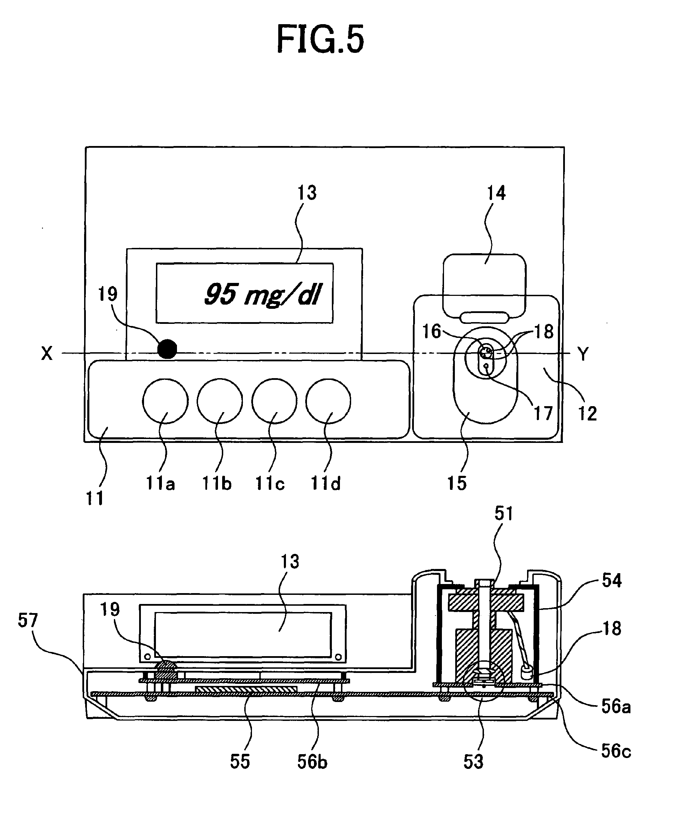

Fig. 5 shows a top plan view and a lateral cross-section of a non-invasive blood sugar level measuring apparatus of an embodiment of the invention. While in this example the skin on the ball of the finger tip is used as the body surface, other parts of the body surface may be used.

On the top surface of the apparatus are provided an operating portion 11, a measuring portion 12 where the finger to be measured is to be placed, and a display portion 13 for displaying the state of the apparatus, measured values, and so on. The operating portion 11 includes four push buttons 11a to 11d for operating the apparatus. The measuring portion 12 has a cover 14 which, when opened (as shown), reveals a finger rest 15 with an oval periphery. The finger rest 15 accommodates an opening end 16 of a radiation temperature sensor portion, a contact temperature sensor portion 17, and an optical sensor portion 18. There are also provided an LED 19 for indicating the state of the apparatus, the measurement timing or the like by way of color, and a buzzer for indicating the state of the apparatus, the measurement timing or the like by way of sound.

The inside of the apparatus will be described by referring to a cross-section thereof. The measuring portion 12 includes a body surface contact portion 51 for the placement of the body surface, a temperature sensor portion 53 for measuring room temperature or the like, and an optical sensor portion 18. The sensors are covered with a sensor case 54, and the sensors and the sensor cover are mounted on a substrate 56a. The display portion and the LED are fixedly mounted on a substrate 56b. A substrate 56c is fixedly mounted on an external casing 57. The substrates 56a and 56b are mounted on the substrate 56c, on which there is further mounted a microprocessor 55 including an arithmetic portion for calculating measurement data and a control portion for centrally controlling individual portions.

Measurement is carried out in the order of pre environment measurement, blood sugar measurement, and post environment measurement. Time allotted for the measurement is 35 sec, 10 sec, and 20 sec, respectively, for example. Blood sugar measurement is conducted while the finger is placed on the finger rest, and post environment measurement is started after the finger has been removed from the finger rest. Thus, if the finger is lifted within five seconds after the measurement of blood sugar, it would take from 65 to 70 seconds between the pre environment measurement and the post environment measurement. During the pre environment measurement and the post environment measurement, chronological changes in room temperature or sensor temperatures, or whether there is any obstacles on the finger rest are monitored.

The measurement environment temperature of the apparatus is then set to be at 20˚C to 28˚C, which is within the range of temperatures in which humans feel comfortable. The skin temperature of man is usually within 33˚C to 35˚C under the aforementioned measurement environment temperature condition. The measurement environment is deemed inappropriate if the radiation temperature sensor, which measures the temperature of the finger, detects temperatures outside a 20˚C to 33˚C range during measurements other than that of blood sugar. The measurement environment is also deemed inappropriate if the range of temperatures of $T_1$ provided by the finger-surface temperature sensor and $T_2$ provided by the heat-conducting portion temperature sensor exceeded the 20˚C to 33˚C temperature range. Further, the measurement environment is deemed improper if the room temperature sensor detects temperatures beyond a temperature range of 20˚C to 28˚C.

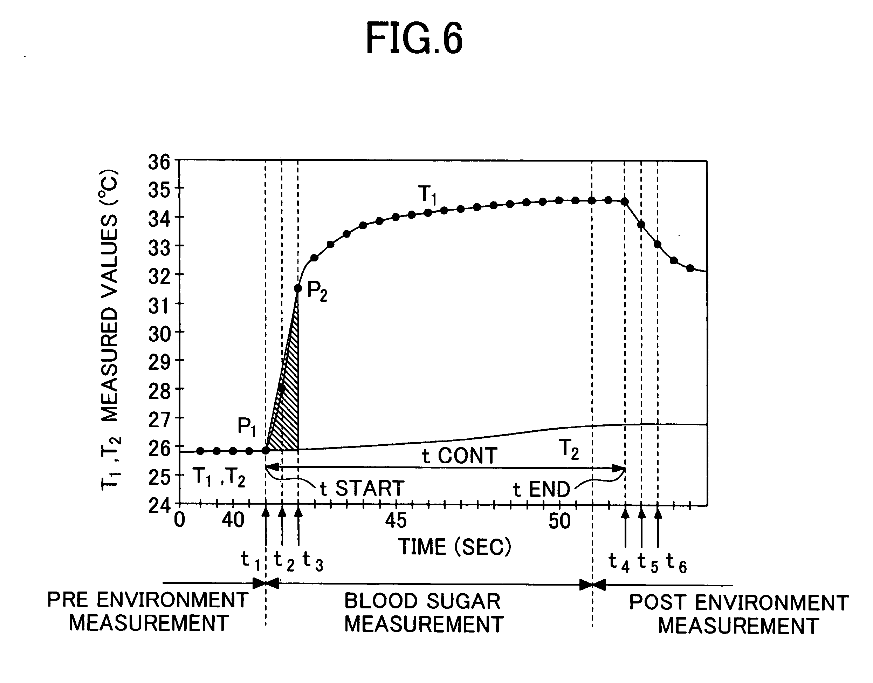

Fig. 6 shows a graph indicating temperature changes in $T_1$ and $T_2$. The dots along the lines are measurement values used for calculation. The amount of change is determined in the following manner, for example. The amount of change in a region corresponding to three measurement points is considered. There are 0.5 second intervals between measurement points, so the distance of three points corresponds to one second. In Fig. 6, the finger is placed at time $t_1$. $T_1$ changes such that it rises sharply between $t_1$ and somewhat later than $t_3$. On the other hand, $T_2$ exhibits hardly any changes in value. Thus, for the purpose of determining the amount of change, the measurement points in $T_2$ corresponding to $t_1$ to $t_3$ are approximated by a straight line and thus $T_2$ is assumed not to change, and the slope of a straight line approximating $T_1$ is taken as suggesting the amount of change. In Fig. 6, $T_1$ at $t_1$ is 25.8˚C, and $T_1$ at $t_3$ is 31.8˚C. Thus, if point $P_1$ is $(t_1, 25.8)$ and point $P_2$ is $(t_3, 31.8)$, the line passing through $P_1$ and $P_2$ is $Y=6X+(31.8-6t_1)$, with the Y axis indicating temperature and the X axis time, and therefore the slope is 6. In this case, $t_1$ is taken as the contact start time $t_{start}$.

As mentioned above, the measurement environment temperature is 20˚C to 28˚C, and the skin temperature is 33˚C to 35˚C, so that the slope is maximum when the environment temperature is 20˚C and the skin temperature is 35˚C, where the slope is 15. The slope is minimum when the environment temperature is 28˚C and the skin temperature is 33˚C, where the slope is 5. Thus, the threshold value of the amount of change $T_1$ that suggests the timing of placement of the finger can be set to be between 5 and 15.

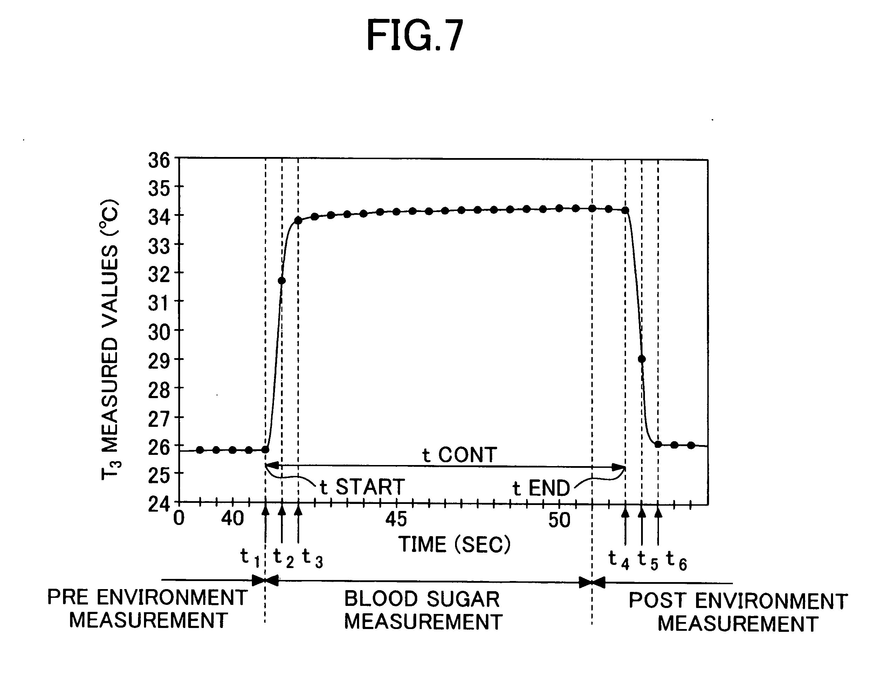

Fig. 7 shows a graph indicating changes in the temperature T3, in which the dots along the line are measurement values used for calculation. The magnitude of the slope at the rise or fall upon the occurrence of temperature differences shows indicates characteristics that differ from one radiation temperature sensor to another. However, by finding a temperature difference in a time interval during which the temperature starts to vary and then becomes steady, such difference can be used as a trigger for one purpose or another.

For example, in Fig. 7, the interval with three dots, namely, $t_4$ to $t_6$, corresponds to the timing in which the value greatly changes and then becomes steady. Thus, temperature changes in this interval with three dots will be considered. When the amount of change in measurement temperature between $t_4$ and $t_6$ is considered, the temperature is 34.1 ˚C at $t_4$ and 26.1 ˚C at $t_6$, producing a difference of 8˚C. The measurement environment temperature range is 20˚C to 28˚C and the skin temperature is 33˚C to 35˚C, so that the range of temperature difference is 5˚C to 15˚C. The example of Fig. 7 falls within this temperature difference range between the measurement environment temperature range and the skin temperature range, and therefore it is determined that the finger has left the finger rest, and then the time of end of contact $t_{end}$ is determined. In this case, $t_4$ is $t_{end}$. Thus, the finger is determined to have left the finger rest when the change in temperature measured by the radiation temperature sensor within a certain time duration falls within a predetermined range, whereupon the time of start of the time duration can be taken as the time of end of contact $t_{end}$. Alternatively, the timing of departure of the finger from the finger rest may be determined to be that point in time when the temperature measured by the radiation temperature sensor started to drop as compared with the previous temperature, and that timing may be taken as the time of end of contact $t_{end}$. Further, the temperature change between $t_1$ and $t_3$ may be used as an auxiliary role for the timing of placement of the finger using the aforementioned $T_1$, thereby improving accuracy for detecting the timing of placement of the finger.

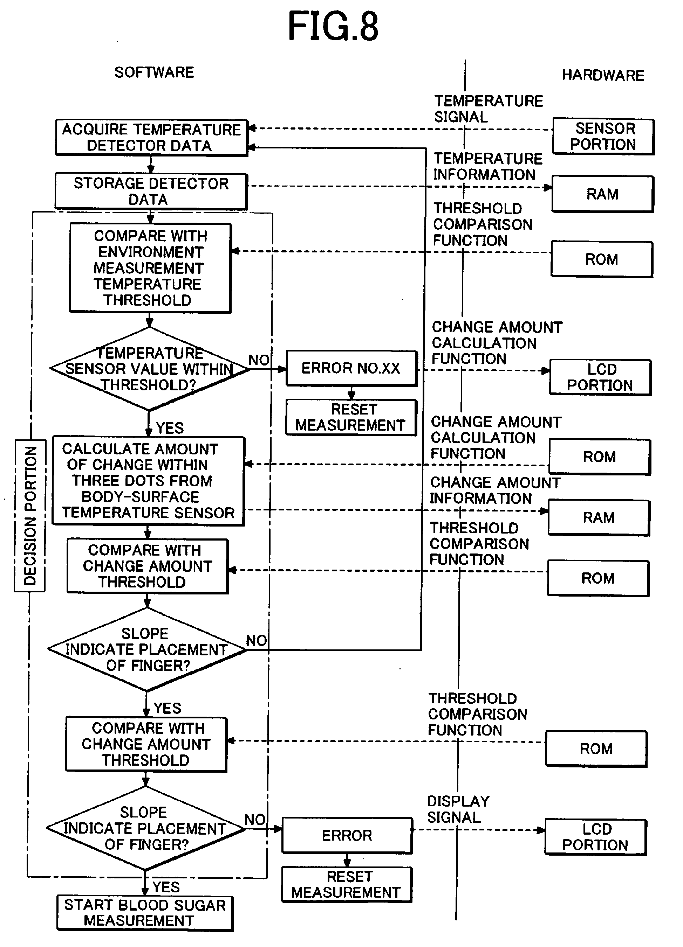

Fig. 8 shows a flowchart illustrating the timing of placement of the finger. The left half of the figure corresponds to the software flow, whereas the right half shows the corresponding hardware elements. A temperature signal is received from the sensor portions, and temperature detector data is acquired and temperature information is stored in RAM. Thereafter, an environment measurement temperature threshold comparing function stored in ROM is calculated in a decision portion of a microcontroller, and it is then determined whether the time change value of the data is within the range of the environment measurement temperature threshold. If it is outside the range, an error is recognized and the measurement is reset and, at the same time, a signal indicating error information is sent to the LCD portion to display an error message. If within the range, the amount of change in the interval corresponding to three dots provided by the body-surface temperature sensor is calculated using a change amount calculation function loaded from ROM onto the decision portion, and the amount of change in the interval corresponding to three dots is compared with the amount of change (slope) in the finger rest using a threshold comparison function loaded from ROM onto the decision portion. If the amount of change in the interval corresponding to three dots is outside the range of the amount of change of the finger rest, temperature detector data is again acquired and the flow is repeated. If the amount of change in the interval corresponding to three dots is within the range, the result of detection provided by the radiation temperature sensor is compared with the finger rest threshold of the radiation temperature sensor, using the threshold comparison function loaded from ROM onto the decision portion. If the result of detection by the radiation temperature sensor is outside the range that indicates the placement of the finger, an error is recognized and the measurement is reset and, at the same time, an error message is displayed on the LCD portion. If within the range, a blood sugar measurement is started.

When deciding on the time of end of contact based on the change in the fall, or drop, of the temperature measured by the radiation temperature sensor, the change amount calculation function and the threshold comparison function or the like can be stored in ROM, as in the above-described step, so that the calculation can be performed in the decision portion based on the result of detection in order to define the timing of departure of the finger. Further, a mechanism may be further provided for displaying the error message or resetting the measurement, as in the above-described step, in case abnormality occurs, such as when, for example, the change in the fall or drop in the aforementioned measured temperature falls outside the predetermined range.

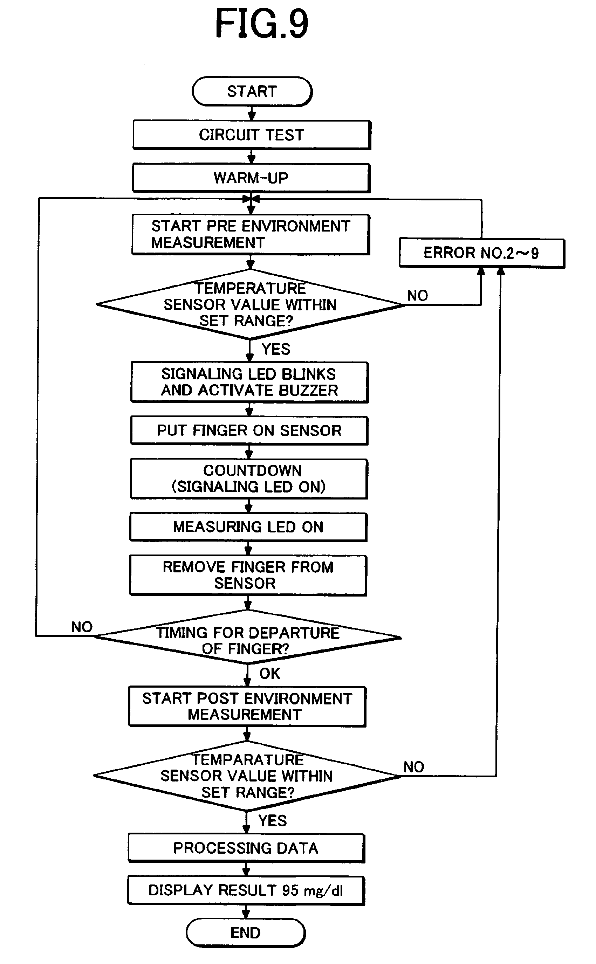

Fig. 9 shows the procedure for operating the apparatus. As one of the buttons on the operating portion is depressed and the apparatus is turned on, an indication "Warming up" is displayed on the LCD portion while the electronic circuits in the apparatus are being warmed up. At the same time, a check program is activated to automatically check the electric circuits. Also, a pre environment measurement is automatically started. Data about the room temperature and sensor temperatures for a certain duration of time is constantly stored in the apparatus. When data that falls outside the set range of any of the temperature detectors is measured, an error is displayed for the particular temperature detector. If the value of the room temperature sensor exceeds an upper limit value, "Error No. 2" is displayed. If the value of the room temperature sensor is below a lower limit value, "Error No. 4" is displayed. If the value of the radiation temperature sensor exceeds an upper limit value, "Error No. 4" is displayed. If the value of the radiation temperature sensor drops below a lower limit value, "Error No. 5" is displayed. Similarly, if the value of the finger surface temperature sensor exceeds an upper limit value, "Error No. 6" is displayed, and if it is below a lower limit value, "Error No. 7" is displayed. Further, if the value of the heat-conducting portion temperature sensor exceeds an upper limit value, "Error No. 8" is displayed, and if the value is below a lower limit value, "Error No. 9" is displayed. If an error occurs, the environment temperature measurement is conducted once again.

If the warm-up phase is over without any error, an indication "Put finger on sensor" appears on the LCD portion. At the same time, the signaling LED mounted on the apparatus either lights or blinks, and the buzzer sounds, thus indicating the completion of measurement preparation. As the user places his or her finger on the finger rest, the LCD portion begins counting down. The signaling LED may be caused to light at the same timing as the countdown. During the countdown, the measuring LED is turned on. When the countdown is over, an indication "Remove finger from sensor" appears on the LCD portion. As the user follows the instruction, the LCD portion indicates "Data calculation." If the finger is separated before the indication "Remove finger from sensor," the LCD portion displays "Do not remove finger from sensor before the countdown is over." If the finger is put away after five seconds or more, the LCD portion displays "Don't remove finger too fast or too slow." Accurate sensor data relating to the measurement of blood sugar cannot be obtained if the finger is put away before the end of countdown. In the present apparatus, a post environment measurement is conducted even after the finger is put away. An error is issued when the finger is put away more than five seconds after the end of countdown because in that case the influence of heat remaining in the heat conducting member becomes large, making it impossible to accurately measure the environment temperature within the post environment measurement time.

As the error message is eliminated using a button, the pre environment measurement is started again, and the LCD portion displays "Put finger on sensor" again, and the measurement process is repeated. If there is no error message, the blood sugar level is displayed on the LCD portion. At this point, the displayed blood sugar level is stored in an IC card, together with the date and time. As the user reads the displayed blood sugar level, he or she pushes another button on the operating portion. About one minute later, the apparatus displays a message "Put finger on sensor" on the LCD portion, thus indicating that the apparatus is ready for the next cycle of measurement.

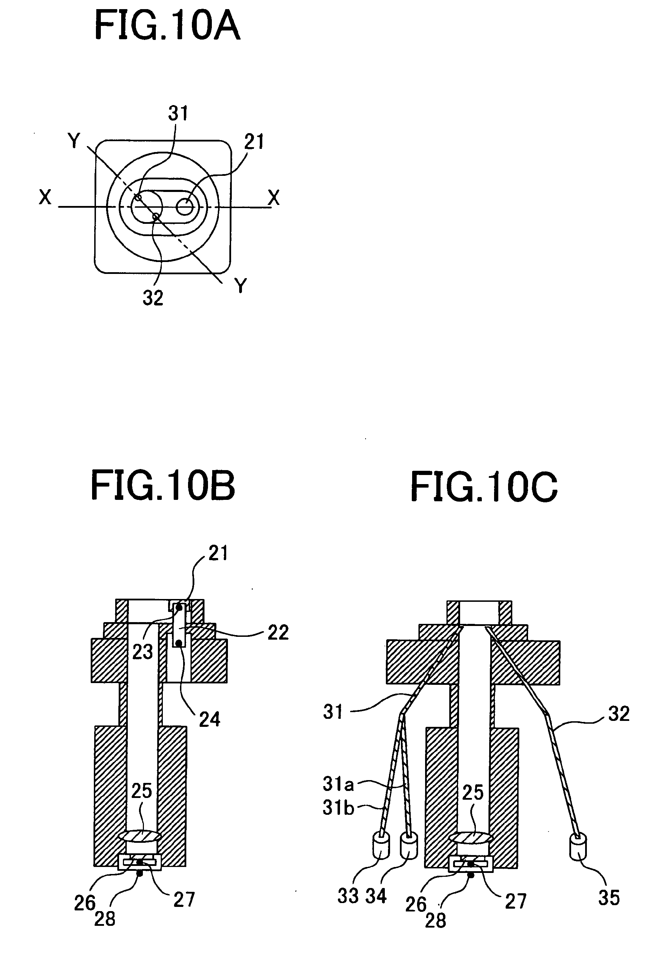

Fig. 10 shows the measuring portion in detail. Fig. 10(a) is a top plan view, (b) is a cross section along line X-X of (a), and (c) is a cross section along line Y-Y of (a).

First, the process of measuring temperature by the non-invasive blood sugar level measuring apparatus according to the invention will be described. In the portion of the measuring portion where the object of measurement (ball of the finger) is to come into contact, a thin plate 21 of a highly heat-conductive material, such as gold, is placed. A bar-shaped heat-conductive member 22 made of material such as polyvinylchloride whose heat conductivity is lower than that of the plate 21 is thermally connected to the plate 21 and extends into the apparatus. The temperature sensors include a thermistor 23 for measuring the temperature of the plate 21 and acting as an adjacent temperature detector with respect to the measured object. There is also a thermistor 24 for measuring the temperature of the heat-conducting member away from the plate 21 by a certain distance and acting as an indirect temperature detector with respect to the measured object. An infrared lens 25 is disposed inside the apparatus at such a position that the measured object (ball of the finger) placed on the finger rest 15 can be seen through the lens. Below the infrared lens 25 is disposed a pyroelectric detector 27 via an infrared radiation-transmitting window 26. Another thermistor 28 is disposed near the pyroelectric detector 27.

Thus, the temperature sensor portion of the measuring portion has four temperature sensors, and they measure four kinds of temperatures as follows:

(1) Temperature on the finger surface (thermistor 23): $T_1$
(2) Temperature of the heat-conducting member (thermistor 24): $T_2$
(3) Temperature of radiation from the finger (pyroelectric detector 27): $T_3$
(4) Room temperature (thermistor 28): $T_4$

The optical sensor portion 18 measures the hemoglobin concentration and the hemoglobin oxygen saturation necessary for the determination of the oxygen supply volume. In order to measure the hemoglobin concentration and the hemoglobin oxygen saturation, absorption must be measured at at least two wavelengths. Fig. 7(c) shows a configuration for carrying out the two-wavelength measurement using two light sources 33 and 34 and one detector 35.

The optical sensor portion 18 includes the ends of two optical fibers 31 and 32. The optical fiber 31 is for optical irradiation, and the optical fiber 32 is for receiving light. As shown in Fig. 7(c), the optical fiber 31 connects to branch fibers 31a and 31b that are provided with light-emitting diodes 33 and 34 at the respective ends thereof. The other end of the light-receiving optical fiber 32 is provided with a photodiode 35. The light-emitting diode 33 emits light with a wavelength of 810 nm, while the light-emitting diode 34 emits light with a wavelength of 950 nm. The wavelength 810 nm is the equal absorption wavelength at which the molar absorbance coefficient of the oxy-hemoglobin is equal to that of the deoxy-hemoglobin. The wavelength 950 nm is the wavelength at which the difference between the molar absorbance coefficient of the oxy-hemoglobin and that of the deoxy-hemoglobin is large.

The two light-emitting diodes 33 and 34 emit light in a time-sharing manner such that the finger of the subject is irradiated with the light emitted by the light-emitting diodes 33 and 34 via the irradiating optical fiber 31. The light shone on the finger is reflected by the skin, enters the light-receiving optical fiber 32, and is eventually detected by the photodiode 35. Part of the light reflected by the skin of the finger penetrates the skin and enters into the tissues and is then absorbed by the hemoglobin in the blood flowing in the capillary blood vessels. The measurement data provided by the photodiode 35 has reflectance R, and the absorbance can be approximately calculated by log(1/R). The finger is thus irradiated with light with the wavelengths of 810 nm and 950 nm, and R is measured for each and also log(1/R) is calculated for each. Thus, absorption $A_1$ and $A_2$ for wavelengths 810 nm and 950 nm, respectively, are measured.

When the deoxy-hemoglobin concentration is [Hb] and the oxy-hemoglobin concentration is [HbO$_2$], absorption $A_1$ and $A_2$ are expressed by the following equations:

$$A_1 = a \times ([Hb] \times A_{Hb}(810nm) + [HbO_2] \times A_{HbO_2}(810nm))$$
$$= a \times ([Hb] + [HbO_2]) \times A_{HbO_2}(810nm)$$

$$A_2 = a \times ([Hb] \times A_{Hb}(950nm) + [HbO_2] \times A_{HbO_2}(950nm))$$
$$= a \times ([Hb] + [HbO_2]) \times ((1 - \frac{[HbO_2]}{[Hb] + [HbO_2]}) \times A_{Hb}(950nm) + \frac{[HbO_2]}{[Hb] + [HbO_2]} \times A_{HbO_2}(950nm))$$

$A_{Hb}$(810 nm) and $A_{Hb}$(950 nm), and $A_{HbO2}$(810 nm) and $A_{HbO2}$(950 nm) are the molar absorbance coefficients of the deoxy-hemoglobin and the oxy-hemoglobin, respectively, and are known at the respective wavelengths. The term a is a proportionality coefficient. The hemoglobin concentration [Hb]+[HbO$_2$], and the hemoglobin oxygen saturation [HbO$_2$]/([Hb]+[HbO$_2$]) can be determined from the above equations as follows:

$$[Hb] + [HbO_2] = \frac{A_1}{a \times A_{HbO_2}(810nm)}$$

$$\frac{[HbO_2]}{[Hb] + [HbO_2]} = \frac{A_2 \times A_{HbO_2}(810nm) - A_1 \times A_{Hb}(950nm))}{A_1 \times (A_{HbO_2}(950nm) - A_{Hb}(950nm))}$$

In the present example, the hemoglobin concentration and the hemoglobin oxygen saturation are measured by measuring absorbance at two wavelengths. Preferably, however, absorbance may be measured at more than two wavelengths so that the influence of interfering components can be reduced and measurement accuracy can be improved.

Fig. 8 shows the concept of how data is processed in the apparatus. The apparatus according to the present example is equipped with five sensors, namely thermistor 23, thermistor 24, pyroelectric detector 27, thermistor 28, and photodiode 35. The photodiode 35 measures absorption at wavelengths 810 nm and 950 nm. Thus, the apparatus is supplied with six kinds of measurement values.

The five kinds of analog signals are supplied via individual amplifiers $A_1$ to As to analog/digital converters $AD_1$ to $AD_5$, where they are converted into digital signals. Based on the digitally converted values, parameters $x_i$ (i=1, 2, 3, 4, 5) are calculated. The following are specific descriptions of $x_i$ (where $a_1$ to $a_5$ are proportionality coefficients):

Parameter proportional to heat radiation

$$x_1 = a_1 \times (T_3)^4$$

Parameter proportional to heat convection

$$x_2 = a_2 \times (T_4 - T_3)$$

Parameter proportional to hemoglobin concentration

$$x_3 = a_3 \times \left( \frac{A_1}{a \times A_{HbO_2}(810nm)} \right)$$

Parameter proportional to hemoglobin saturation

$$x_4 = a_4 \times \left( \frac{A_2 \times A_{HbO_2}(810nm) - A_1 \times A_{Hb}(950nm))}{A_1 \times (A_{HbO_2}(950nm) - A_{Hb}(950nm))} \right)$$

Parameter proportional to blood flow volume

$$x_5 = a_5 \times \left( \frac{1}{t_{CONT} \times (S_1 - S_2)} \right)$$

Then, normalized parameters are calculated from mean values and standard deviations of $x_i$ obtained by actual data pertaining to large numbers of able-bodied people and diabetic patients. A normalized parameter $X_i$ (where i=1, 2, 3, 4, 5) is calculated from each parameter $x_i$ according to the following equation:

$$X_i = \frac{x_i - \bar{x}_i}{SD(x_i)}$$

where

$x_i$: parameter
$\bar{x}_i$ : mean value of the parameter
$SD(x_i)$: standard deviation of the parameter

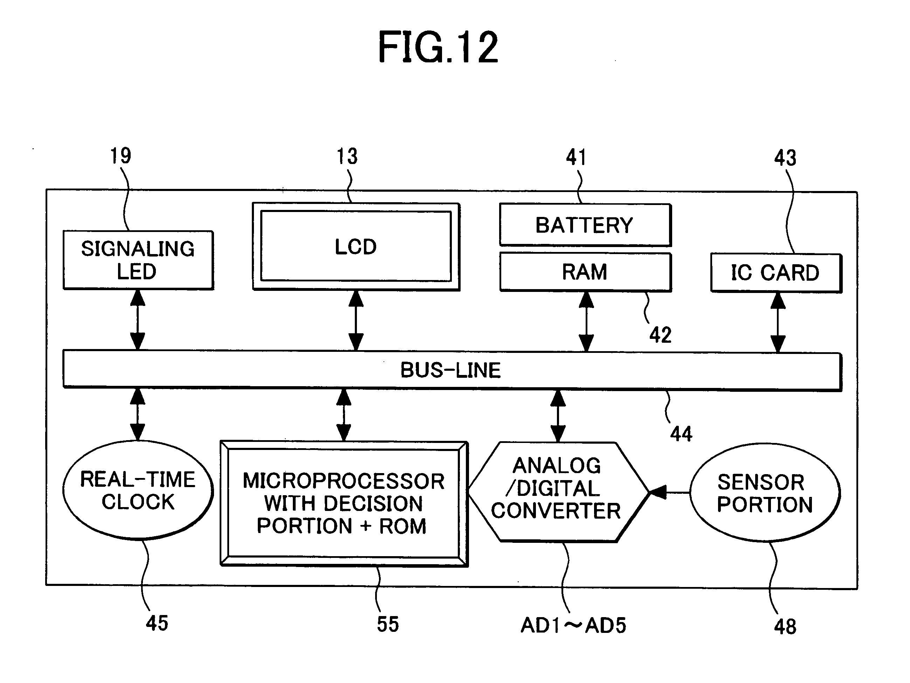

Using the above five normalized parameters, calculations are conducted for conversion into glucose concentration to be eventually displayed. Fig. 12 shows an example of the internal configuration of the apparatus. An LCD 13 and a signaling LED 19 are disposed at such positions that they can be within the field of view of the user when the finger is placed on a sensor portion 48. A program necessary for the processing calculations is stored in a ROM in the microprocessor built inside the apparatus. The memory region required for the processing calculations is ensured in a RAM similarly built inside the apparatus. The analog signal in the sensor portion is converted by analog/digital converters AD1 to AD5 into a digital signal, which is then transferred via a bus line 44 to a microprocessor where it is processed using a function stored in ROM. Depending on the result of calculations, a signaling LED 19 either lights up or blinks. If the signal from the sensor indicates that the finger is placed on the finger rest, the LCD portion displays a countdown under the instruction of a real time clock 45 and, at the same time, a blood sugar measuring program stored in ROM is started. The result of calculations is displayed on the LCD portion and may be simultaneously stored in an IC card 43. When battery 41 runs low, a warning may be displayed on the LCD portion, or the signaling LED may be caused to light or blink.

The details of the relationship between the measuring flow and the circuit configuration will be described. During a pre environment measurement, the sensor value in the form of a digital signal obtained by the analog/digital converter is computed in the microprocessor into temperature data. When an environment temperature range error occurs, a message is displayed on the LCD, and a warning is issued via the signaling LED or the buzzer. If there is no error, the data is temporarily stored in RAM. If the data stored in RAM exceeds the amount corresponding to the time of pre environment measurement, the older data is deleted such that the data stored in RAM is always that of the amount corresponding to the pre environment measurement. Of the 35 seconds of $T_1$ data, the slope of the latest 1-second portion (3 data) is always calculated, and if the slope is within the aforementioned range of threshold, it is determined that the finger has touched the finger rest, and the subsequent data is stored in RAM as blood sugar measurement value data. After the end of countdown, if the $T_3$ value indicates that the finger has left the finger rest, the following 20 seconds of data is stored in RAM as post environment measurement data. After all of the measurements are over, the microprocessor calculates the blood sugar level using the blood sugar level calculation function stored in ROM. The microprocessor then writes the blood sugar level in RAM and in an IC card. During this series of processes, the microprocessor sends signals to the signaling LED, the buzzer and the LCD as necessary, so that the user can be informed of an error message, the state of progress, or the result of measurement.

The ROM stores, as a constituent element of the program necessary for the processing calculations, a function for determining glucose concentration C in particular. The function is defined as follows. C is expressed by the below-indicated equation (1), where $a_i$ (i=0, 1, 2, 3, 4, 5) is determined from a plurality of pieces of measurement data in advance according to the following procedure:

(1) A multiple regression equation is created that indicates the relationship between the normalized parameter and the glucose concentration C.
(2) A normalized equation (simultaneous equation) relating to the normalized parameter is obtained from an equation obtained by the least-squares method.
(3) Values of coefficient $a_i$ (i=0, 1, 2, 3, 4, 5) are determined from the normalized equation and then substituted into the multiple regression equation.

Initially, the regression equation (1) indicating the relationship between the glucose concentration C and the normalized parameters $X_1$, $X_2$, $X_3$, $X_4$, and $X_5$ is formulated.

$$C = f(X_1, X_2, X_3, X_4, X_5)$$
$$= a_0 + a_1 X_1 + a_2 X_2 + a_3 X_3 + a_4 X_4 + a_5 X_5 \quad \dots\dots(1)$$

Then, the least-squares method is employed to obtain a multiple regression equation that would minimize the error with respect to a measured value Ci of glucose concentration according to an enzyme electrode method. When the sum of squares of the residual is D, D is expressed by the following equation (2):

$$D = \sum_{i=1}^{n} d_i^2$$

$$= \sum_{i=1}^{n} (C_i - f(X_{i1}, X_{i2}, X_{i3}, X_{i4}, X_{i5}))^2$$

$$= \sum_{i=1}^{n} \{C_i - (a_0 + a_1 X_{i1} + a_2 X_{i2} + a_3 X_{i3} + a_4 X_{i4} + a_5 X_{i5})\}^2 \quad \dots\dots(2)$$

Because the sum of squares of the residual D becomes minimum when partial differentiation of equation (2) with respect to $a_0$, $a_2$,..., $a_5$ gives zero. Thus, we have the following equations:

$$\frac{\partial D}{\partial a_0} = -2 \sum_{i=1}^{n} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_1} = -2 \sum_{i=1}^{n} X_{i1} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_2} = -2 \sum_{i=1}^{n} X_{i2} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_3} = -2 \sum_{i=1}^{n} X_{i3} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_4} = -2 \sum_{i=1}^{n} X_{i4} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_5} = -2 \sum_{i=1}^{n} X_{i5} \{C_i - (a_0 + a_1 X_{i1} + a_2 X_{i2} + a_3 X_{i3} + a_4 X_{i4} + a_5 X_{i5})\} = 0 \quad \dots\dots(3)$$

When the mean values of C and $X_1$ to $X_5$ are $C_{mean}$ and $X_{1mean}$ to $X_{5mean}$, respectively, since $X_{imean}=0$ (i=1 to 5), equation (4) can be obtained from equation (1) thus:

$$a_0 = C_{mean} - a_1 X_{1mean} - a_2 X_{2mean} - a_3 X_{3mean} - a_4 X_{4mean} - a_5 X_{5mean}$$
$$= C_{mean} \qquad \qquad \qquad ......(4)$$

The variation and covariation between the normalized parameters are expressed by equation (5). Covariation between the normalized parameter $X_i$ (i=1 to 5) and C is expressed by equation (6).

$$S_{ij} = \sum_{k=1}^{n}(X_{ki} - X_{imean})(X_{kj} - X_{jmean}) = \sum_{k=1}^{n} X_{ki} X_{kj} \quad (i, j = 1,2,..5) \quad ......(5)$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,..5) \quad ......(6)$$

Substituting equations (4), (5), and (6) into equation (3) and rearranging yields simultaneous equation (normalized equation) (7). Solving equation (7) yields $a_1$ to $a_5$.

$$a_1 S_{11} + a_2 S_{12} + a_3 S_{13} + a_4 S_{14} + a_5 S_{15} = S_{1C}$$

$$a_1 S_{21} + a_2 S_{22} + a_3 S_{23} + a_4 S_{24} + a_5 S_{25} = S_{2C}$$

$$a_1 S_{31} + a_2 S_{32} + a_3 S_{33} + a_4 S_{34} + a_5 S_{35} = S_{3C}$$

$$a_1 S_{41} + a_2 S_{42} + a_3 S_{43} + a_4 S_{44} + a_5 S_{45} = S_{4C}$$

$$a_1 S_{51} + a_2 S_{52} + a_3 S_{53} + a_4 S_{54} + a_5 S_{55} = S_{5C} \quad ......(7)$$

Constant term $a_0$ is obtained by means of equation (4). The thus obtained $a_i$ (i=0, 1, 2, 3, 4, 5) is stored in ROM at the time of manufacture of the apparatus. In actual measurement using the apparatus, the normalized parameters $X_1$ to $X_5$ obtained from the measured values are substituted into regression equation (1) to calculate the glucose concentration C.

Hereafter, an example of the process of calculating the glucose concentration will be described. The coefficients in equation (1) are determined in advance based on large data obtained from able-bodied persons and diabetic patients. The ROM in the microprocessor stores the following formula for the calculation of glucose concentration:

$$C = 99.4 + 18.3 \times X_1 - 20.2 \times X_2 - 23.7 \times X_3 - 22.0 \times X_4 - 25.9 \times X_5$$

$X_1$ to $X_5$ are the results of normalization of parameters $x_1$ to $x_5$. Assuming the distribution of the parameters is normal, 95% of the normalized parameter takes on values between -2 to +2.

In the case of an able-bodied person, substituting exemplary measurement values in the above equation such that $X_1$=-0.06, $X_2$=+0.04, $X_3$=+0.05, $X_4$=-0.12, and $X_5$=+0.10 yields C=96 mg/dl. In the case of a diabetic patient, substituting exemplary measurement values in the equation such that $X_1$=+1.15, $X_2$=-1.02, $X_3$=-0.83, $X_4$=-0.91, and $X_5$=-1.24 yields C=213 mg/dl.

Hereafter, the results of measurement by the conventional enzymatic electrode method and those by an embodiment of the invention will be compared. In the enzymatic electrode method, a blood sample is reacted with a reagent and the amount of resultant electrons is measured to determine glucose concentration. When the glucose concentration for an able-bodied person was 89 mg/dl according to the enzymatic electrode method, the normalized parameters obtained by measurement at the same time according to the invention were $X_1$=-0.06, $X_2$=+0.04, $X_3$=+0.05, $X_4$=-0.12, and $X_5$=+0.10. Substituting these values into the above equation yields C=96 mg/dl. On the other hand, when the glucose concentration for a diabetic patient was 238 mg/dl according to the enzymatic electrode method, the normalized parameters obtained by measurement at the same time according to the invention were $X_1$=+1.15, $X_2$=-1.02, $X_3$=-0.83, $X_4$=-0.91, and $X_5$=-1.24. Substituting these values into the above equation yields C=213 mg/dl. The results thus indicated that the invention can provide highly accurate glucose concentration.

Fig. 9 shows the plot of glucose concentration for a plurality of patients. The calculated values of glucose concentration according to the invention are shown on the vertical axis, and the measured values of glucose concentration according to the enzymatic electrode method are shown on the horizontal axis. It will be seen that a good correlation can be obtained by measuring the oxygen supply volume and the blood flow volume according to the method of the invention (correlation coefficient=0.9324).

Thus, the invention can provide a highly accurate non-invasive blood sugar level measuring apparatus.

**Claims**

1. A blood sugar level measuring apparatus comprising:

   a body surface contact portion (15, 51);
   a temperature detecting portion (16,17,53) for measuring the temperature on a body surface and environment temperature;
   an optical measuring portion (18) for measuring hemoglobin concentration and hemoglobin oxygen saturation in blood;
   a calculation portion (55) for calculating blood sugar level based on measurement data provided by said temperature detecting portion and measurement data provided by said optical measuring portion;
   a display portion (13, 19); and
   a control portion (55) for centrally controlling individual portions,

   **characterized by**:

   an error decision portion (55) for determining whether either the temperature on said body surface or the environment temperature is within a predetermined range, wherein said control portion is adapted to:

      cause said calculation portion to calculate said blood sugar level if said error decision portion determines that the temperature on said body surface and said environment temperature are within said predetermined range,
      identify the moment of contact of the body surface to said body surface contact portion (15,51) and the moment of departure of the body surface from said body surface contact portion by detecting chronological changes in the output from said temperature detecting portion, wherein, upon detection of contact of the body surface to said body surface contact portion, said measurement data is stored and an advance-notice display is made on said display portion (13,19) indicating the timing of departure of the body surface from said body surface contact portion, and
      instruct an error display to be made on said display portion (13, 19) and reset the measurement upon detection of the departure of the body surface from said body surface contact portion before said timing is reached, or in the absence of detection of the departure of the body surface from said body surface contact portion even after a predetermined time following said timing.

2. A blood sugar level measuring apparatus according to claim 1, wherein the advance-notice display of the timing of departure of the body surface from said body surface contact portion is made by way of a countdown displayed on said display portion (13).

3. A blood sugar level measuring apparatus according to claim 1 or 2, wherein said error decision portion is adapted to determine whether the output from said temperature detecting portion is within a preset range when the body surface is not in contact with said body surface contact portion, wherein an error display is made and the measurement is reset if said output is outside the predetermined range.

**Patentansprüche**

1. Blutzuckerpegel-Meßvorrichtung, aufweisend
   einen Körperoberflächen-Berührungsabschnitt (15, 51),
   einen Temperaturerfassungsabschnitt (16, 17, 53) zur Messung der Temperatur auf der Körperoberfläche und der Umgebungstemperatur,
   einen optischen Meßabschnitt (18) zur Messung der Haemoglobin-Konzentration und der Haemoglobin-Sauerstoffsättigung im Blut,
   einen Berechnungsabschnitt (55) zum Berechnen des Blutzuckerpegels aufgrund von vom Temperaturerfassungsabschnitt gelieferten Meßdaten und vom optischen Meßabschnitt gelieferten Meßdaten,
   einen Anzeigeabschnitt (13, 19), und
   einen Steuerabschnitt (55) zum zentralen Steuern der einzelnen Abschnitte,
   **gekennzeichnet durch**
   einen Fehlerbeurteilungsabschnitt (55) zum Bestimmen, ob entweder die Temperatur auf der Körperoberfläche oder die Umgebungstemperatur innerhalb eines vorbestimmten Bereichs liegen, wobei der Steuerabschnitt eingerichtet ist,
   den Berechnungsabschnitt zu veranlassen, den Blutzuckerpegel zu berechnen, wenn der Fehlerbeurteilungsabschnitt bestimmt, daß die Temperatur auf der Körperoberfläche und die Umgebungstemperatur innerhalb des vorbestimmten Bereichs liegen,
   den Zeitpunkt einer Berührung des Körperoberflächen-Berührungsabschnitts (15, 51) **durch** die Körperoberfläche und den Zeitpunkt des Lösens der Körperoberfläche vom Körperoberflächen-Berührungsabschnitt **durch** aufeinanderfolgende Änderungen in der Ausgabe des Temperaturerfassungsabschnitts zu erkennen, wobei aufgrund einer Erfassung einer Berührung des Körperoberflächen-Berührungsabschnitts **durch** die Körperoberfläche die Meßdaten gespeichert werden und auf dem Anzeigeabschnitt (13, 19) eine Vorab-Nachricht angezeigt wird, die die Zeit des Lösens der Körperoberfläche vom Körperoberflächen-Berührungsabschnitt angibt, und
   auf dem Anzeigeabschnitt (13, 19) die Anzeige eines Fehlers zu veranlassen und die Messung zurückzusetzen, sobald vor Erreichen der genannten Zeit das Lösen der Körperoberfläche vom Körperoberflächen-Berührungsabschnitt erfaßt wird oder wenn auch nach einer vorbestimmten Zeitspanne im Anschluß an diese Zeit das Lösen der Körperoberfläche vom Körperoberflächen-Berührungsabschnitt nicht erfaßt wird.

2. Vorrichtung nach Anspruch 1, wobei die Anzeige der Vorab-Nachricht der Zeit des Lösens der Körperoberfläche vom Körperoberflächen-Berührungsabschnitt mittels eines Rückwärtszählens vorgenommen wird, das auf dem Anzeigeabschnitt (13) angezeigt wird.

3. Vorrichtung nach Anspruch 1 oder 2, wobei der Fehlerbeurteilungsabschnitt eingerichtet ist zu erfassen, ob die Ausgabe des Temperaturerfassungsabschnitts innerhalb eines voreingestellten Bereichs liegt, wenn die Körperoberfläche den Körperoberflächen-Berührungsabschnitt nicht berührt, wobei eine Fehleranzeige erfolgt und die Messung zurückgesetzt wird, wenn sich die Ausgabe außerhalb des vorbestimmten Bereichs befindet.

**Revendications**

1. Dispositif de mesure de taux de glycémie comportant :

   une partie de contact avec une surface du corps (15, 51),
   une partie de détection de température (16, 17, 53) pour mesurer la température sur une surface du corps et une température d'environnement,
   une partie de mesure optique (18) pour mesurer la concentration en hémoglobine et la saturation en oxygène de l'hémoglobine dans le sang,
   une partie de calcul (55) pour calculer un taux de glycémie sur la base de données de mesure délivrées par ladite partie de détection de température et de données de mesure délivrées par ladite partie de mesure optique,
   une partie d'affichage (13, 19), et
   une partie de commande (55) pour commander de manière centrale des parties individuelles,

   **caractérisé par** :

   une partie de détermination d'erreur (55) pour déterminer si la température sur ladite surface du corps ou la température d'environnement se trouve dans une plage prédéterminée, ladite partie de commande étant adaptée pour :

amener ladite partie de calcul à calculer ledit taux de glycémie si ladite partie de détermination d'erreur détermine que la température sur ladite surface du corps et ladite température d'environnement se trouvent dans ladite plage prédéterminée,

identifier le moment de contact de la surface du corps avec la partie de contact avec une surface du corps (15, 51) et le moment de l'éloignement de la surface du corps de ladite partie de contact avec une surface du corps en détectant des changements chronologiques dans la sortie de ladite partie de détection de température, dans lequel, lors de la détection d'un contact entre la surface du corps et ladite partie de contact avec une surface du corps, lesdites données de mesure sont mémorisées et un affichage de notification à l'avance est réalisé sur ladite partie d'affichage (13, 19) en indiquant le moment d'éloignement de la surface du corps de ladite partie de contact avec une surface du corps, et

ordonner qu'un affichage d'erreur soit réalisé sur ladite partie d'affichage (13, 19) et réinitialiser la mesure lors d'une détection de l'éloignement de la surface du corps de ladite partie de contact avec une surface du corps avant que ledit instant soit atteint, ou en l'absence de détection de l'éloignement de la surface du corps de la partie de contact avec une surface du corps même après un temps prédéterminé après ledit instant.

2. Dispositif de mesure de taux de glycémie selon la revendication 1, dans lequel l'affichage de notification à l'avance de l'instant d'éloignement de la surface du corps de ladite partie de contact avec une surface du corps est réalisé par un décompte affiché sur ladite partie d'affichage (13).

3. Dispositif de mesure de taux de glycémie selon la revendication 1 ou 2, dans lequel ladite partie de détermination d'erreur est adaptée pour déterminer si la sortie de ladite partie de détection de température est dans une plage prédéfinie lorsque la surface du corps n'est pas en contact avec ladite partie de contact avec une surface du corps, dans lequel un affichage d'erreur est effectué et la mesure est réinitialisée si ladite sortie est en dehors de la plage prédéterminée.

# FIG.1

# FIG.2

# FIG.3

# FIG.4

CONTACT TEMPERATURE $T_1$, $T_2$

RADIATION TEMPERATURE $T_3$

ROOM TEMPERATURE $T_4$

HEMOGLOBIN ABSORBANCE $A_1$

HEMOGLOBIN ABSORBANCE $A_2$

AMOUNT OF CONVECTIVE HEAT TRANSFER

AMOUNT OF RADIATION HEAT TRANSFER

AMOUNT OF HEAT DISSIPATION

BLOOD FLOW VOLUME

HEMOGLOBIN CONCENTRATION

HEMOGLOBIN OXYGEN SATURATION

VOLUME OF OXYGEN SUPPLY

# FIG.5

95 mg/dl

FIG.6

# FIG.7

EP 1 518 493 B1

# FIG.8

SOFTWARE

HARDWARE

TEMPERATURE
SIGNAL

```
┌──────────────────────┐          SENSOR
│ ACQUIRE TEMPERATURE  │◄ - - - - PORTION
│   DETECTOR DATA      │◄───
└──────────────────────┘     TEMPERATURE
          │                  INFORMATION
          ▼
┌──────────────────────┐
│  STORAGE DETECTOR    │ - - - -      RAM
│       DATA           │
└──────────────────────┘     THRESHOLD
          │                  COMPARISON
          ▼                  FUNCTION
┌──────────────────────┐
│   COMPARE WITH       │
│   ENVIRONMENT        │◄ - - - -     ROM
│   MEASUREMENT        │
│   TEMPERATURE        │
│   THRESHOLD          │
└──────────────────────┘
          │                  CHANGE AMOUNT
          ▼                  CALCULATION
     ╱TEMPERATURE╲           FUNCTION
   ╱ SENSOR VALUE WITHIN╲ NO ┌──────────────┐      LCD
   ╲  THRESHOLD?        ╱───►│  ERROR NO.XX │ - - -PORTION
     ╲          ╱           └──────────────┘
          │ YES                    │
          ▼                        ▼
┌──────────────────────┐   ┌──────────────┐ CHANGE AMOUNT
│ CALCULATE AMOUNT     │   │    RESET     │ CALCULATION
│ OF CHANGE WITHIN     │   │ MEASUREMENT  │ FUNCTION
│ THREE DOTS FROM      │◄ -└──────────────┘- -     ROM
│ BODY-SURFACE         │
│ TEMPERATURE SENSOR   │ - - - - CHANGE AMOUNT
└──────────────────────┘        INFORMATION
          │                                         RAM
          ▼                  THRESHOLD
┌──────────────────────┐     COMPARISON
│   COMPARE WITH       │     FUNCTION
│   CHANGE AMOUNT      │◄ - - - -     ROM
│   THRESHOLD          │
└──────────────────────┘
          │
          ▼
      ╱ SLOPE ╲
   ╱ INDICATE PLACEMENT╲ NO
   ╲  OF FINGER?       ╱───┘
     ╲          ╱
          │ YES             THRESHOLD
          ▼                 COMPARISON
┌──────────────────────┐    FUNCTION
│   COMPARE WITH       │
│   CHANGE AMOUNT      │◄ - - - -     ROM
│   THRESHOLD          │
└──────────────────────┘
          │
          ▼                 DISPLAY
      ╱ SLOPE ╲              SIGNAL
   ╱ INDICATE PLACEMENT╲ NO ┌──────────────┐      LCD
   ╲  OF FINGER?       ╱───►│    ERROR     │ - - -PORTION
     ╲          ╱           └──────────────┘
          │ YES                    │
          ▼                        ▼
┌──────────────────────┐   ┌──────────────┐
│ START BLOOD SUGAR    │   │    RESET     │
│   MEASUREMENT        │   │ MEASUREMENT  │
└──────────────────────┘   └──────────────┘
```

DECISION PORTION

# FIG.9

```
              ┌─────────────┐
              │    START    │
              └─────────────┘
                     │
         ┌───────────────────────┐
         │     CIRCUIT TEST      │
         └───────────────────────┘
                     │
         ┌───────────────────────┐
         │       WARM-UP         │
         └───────────────────────┘
                     │
         ┌───────────────────────┐
         │ START PRE ENVIRONMENT │
         │     MEASUREMENT       │
         └───────────────────────┘
                     │
              ╱TEMPERATURE╲
            ╱ SENSOR VALUE WITHIN ╲──NO──►  ┌──────────────┐
            ╲   SET RANGE?      ╱           │ ERROR NO.2~9 │
              ╲             ╱               └──────────────┘
                   │ YES
         ┌───────────────────────┐
         │ SIGNALING LED BLINKS  │
         │  AND ACTIVATE BUZZER  │
         └───────────────────────┘
                     │
         ┌───────────────────────┐
         │  PUT FINGER ON SENSOR │
         └───────────────────────┘
                     │
         ┌───────────────────────┐
         │      COUNTDOWN        │
         │  (SIGNALING LED ON)   │
         └───────────────────────┘
                     │
         ┌───────────────────────┐
         │    MEASURING LED ON   │
         └───────────────────────┘
                     │
         ┌───────────────────────┐
         │  REMOVE FINGER FROM   │
         │       SENSOR          │
         └───────────────────────┘
                     │
    NO ◄─────╱TIMING FOR DEPARTURE╲
             ╲    OF FINGER?     ╱
                   │ OK
         ┌───────────────────────┐
         │ START POST ENVIRONMENT│
         │     MEASUREMENT       │
         └───────────────────────┘
                     │
              ╱TEMPARATURE╲
            ╱ SENSOR VALUE WITHIN ╲──NO──►
            ╲   SET RANGE?      ╱
                   │ YES
         ┌───────────────────────┐
         │    PROCESSING DATA    │
         └───────────────────────┘
                     │
         ┌───────────────────────┐
         │ DISPLAY RESULT 95 mg/dl│
         └───────────────────────┘
                     │
              ┌─────────────┐
              │     END     │
              └─────────────┘
```

# FIG.10A

# FIG.10B

# FIG.10C

# FIG.11

23 → A1 → AD1 →

24 → A2 → AD2 →

27 → A3 → AD3 → CONVERSION FROM TEMPERATURE MEASUREMENT DATA INTO NORMALIZED PARAMRTERS ⟹

28 → A4 → AD4 →

810nm 950nm
35 → A5 → AD5 → CONVERSION FROM OPTICAL MEASUREMENT DATA INTO NORMALIZED PARAMETERS ⟹

CONVERSION FROM NORMALIZED PARAMETERS INTO GLUCOSE CONCENTRATION

EP 1 518 493 B1

# FIG.12

SIGNALING LED — 19

LCD — 13

BATTERY — 41

RAM — 42

IC CARD — 43

BUS-LINE — 44

REAL-TIME CLOCK — 45

MICROPROCESSOR WITH DECISION PORTION + ROM — 55

ANALOG /DIGITAL CONVERTER — AD1〜AD5

SENSOR PORTION — 48

EP 1 518 493 B1

# FIG.13

COMPARATIVE METHOD
(ENZYMATIC ELECTRODE)